# EUROPEAN PATENT SPECIFICATION

(11) **EP 1 490 100 B1**
(45) Date of publication and mention of the grant of the patent: **28.12.2011**
(21) Application number: 03720397.3
(22) Date of filing: 28.03.2003
(51) Int. Cl.: A61K 39/00, A61K 39/39, A61K 48/00

(54) **COMBINED DNA/PROTEIN VACCINE COMPOSITIONS**
KOMBINIERTE DNS/PROTEIN IMPFSTOFFZUSAMMENSETZUNGEN
COMPOSITIONS DE VACCINS A PROTEINES/ADN COMBINES

(30) Priority: 28.03.2002 EP 02076276; 10.02.2003 EP 03075383
(43) Date of publication of application: 29.12.2004
(73) Proprietor: BRENNTAG BIOSECTOR A/S, 3600 Frederikssund (DK)
(72) Inventor: REIMANN, Hansjörg, 89081 Ulm-Lehr (DE); SCHIRMBECK, Reinhold, Alois, 36381 Krumbach (DE); LINDBLAD, Erik, B., DK-1820 Frederiksberg (DK)
(74) Representative: De Clercq, Ann G. Y.
(86) International application number: PCT/EP2003/003353
(87) International publication number: WO 2003/082327

(56) References cited:
- WO-A-00/02591
- WO-A-00/68259
- WO-A-93/24148
- WO-A-97/28818
- WO-A-99/30733
- S. WANG ET AL.: "Enhanced type I immune response to a hepatitis B DNA vaccine by formulation with calcium- or aluminium phosphate." VACCINE, vol. 18, no. 13, 21 January 2000 (2000-01-21), pages 1227-1235, XP002234919 Guildford, GB cited in the application

## Description

### Field of the invention

The present invention relates to novel, combined DNA/Protein vaccine compositions comprising at least one nucleic acid molecule encoded antigen and at least one protein antigen adjuvanted in a mineral-based adjuvant so as to enhance the immunogenicity of both in a single vaccine formulation.

### Background of the invention

DNA vaccination is an important novel immunization strategy to specifically prime humoral and cellular immune responses (S. Gurunathan et al., Annu Rev. Immunol. (2000) 18:927-74). With DNA vaccines based on injection of nucleotides alone 50-100 µg nucleotide is required to elicit a potent immune response against the antigen encoded by the DNA. It is not known how large a proportion of the DNA that is *de facto* transcribed into protein and it is not clear how much protein that it subsequently gives lead to. It has been shown, however, that no or barely detectable immune responses are detected when <10 µg plasmid DNA is injected into mice.

Currently, DNA vaccines are not yet on the market. Limitations that prevent the introduction of DNA vaccination into clinical practice include the conspicious absence of techniques that can deliver low doses of a DNA vaccine in a sufficiently immunogenic form to a wide range of animals and to man. It will be clear that the development of techniques which would lead to the elicitation of a potent immune response after administration of significantly lower doses of DNA nucleotide would attract both scientific and economic interest among vaccine producers.

In recent literature various adjuvants have been proposed to enhance the immunogenicity of DNA vaccines. For example, the interrelated references WO 00/02591, WO 98/35562, and S. Wang et al., Vaccine 18:1227-1235 (2000) disclose vaccine formulations comprising nucleic acid molecules and an aluminium phosphate-based or calcium phosphate-based adjuvant provided in a biologically effective concentration so as to improve induction of an immune response subsequent to vaccination. The formulations with calcium- or aluminum phosphate adjuvants prior to injection have been shown to facilitate delivery of DNA vaccines, since antibody titers are increased by 10- to 100-fold and the immunogenic dose of DNA is decreased by 10-fold. Although the mechanism by which aluminium phosphate or aluminium hydroxyphosphate exerts this adjuvant effect is not clear, the adjuvants have an extensive record from use in practical vaccination.

WO 99/51269 discloses a DNA vaccine containing a naked DNA incorporating and expressing *in vivo* a nucleotide sequence encoding an antigenic polypeptide and at least an adjuvant compound selected from the class of (meth)acrylic polymers and copolymers of maleic anhydride and alkenyl derivatives, preferably a carbomer or an EMA®.

WO 02/03961 discloses a nucleic acid delivery system comprising nucleic acid molecules encapsulated in biodegradable microspheres, suitable for delivering DNA vaccines, which comprises an adjuvant for modulating the immunostimulatory efficacy said adjuvant comprising an aminoalkyl glucoaminide-4-phosphate (AGP).

WO99/30733 discloses a method to enhance an immune response based on a combination of polynucleotide and polypeptide. Pharmaceutical compositions comprising DNA + polypeptide may contain a specially prepared aluminium hydroxide (Al(OH)₃). The protein antigen was adsorbed onto aluminium hydroxide (Example 8) and subsequently co-acervated with polycaprolactone to create a complex form with slow-release of antigen from the specially treated Al(OH)₃. This complex was then mixed with the DNA in the ratio 2 µg protein to 10 µg DNA prior to use. No other approaches to adjuvantation of the peptide-nucleotide combination using other mineral-based adjuvants were investigated.

WO 97/28818 discloses vaccinations and vaccines comprising a nucleic acid encoding a first epitope and a peptide encoding a second epitope, in particular *in vitro* transfection of APC's (Antigen Presenting Cells) with DNA encoded antigen (and not muscular tissue which is the normal target tissue for DNA immunization) and the simultaneous presence of peptide antigen as well as DNA encoded and transcribed antigen in APC's was demonstrated. Several vectors and delivery systems were discussed and a speculation was made (p. 42) on the possible use of a wide range of adjuvants, but no experimental data for the use of any adjuvant was provided.

WO00/68259 discloses the use of bovine serum albumine as a model protein antigen, but does not refer to a vaccine composition comprising a polynucleotide vaccine component and a protein antigen vaccine component.

WO93/24148 relates to combined vaccines comprising hepatitis B surface antigen (HBsAg) and other antigens, in which HBsAg is adsorbed to aluminium phopshate or aluminium hydroxide. However, WO93/24148 does not relate to a vaccine composition comprising a polynucleotide vaccine component and a protein antigen vaccine component.

Conventional vaccines allow the combination of different antigens, but only within the group of either adjuvanted vaccine components or within the group of non-adjuvanted live-attenuated vaccine relevant organisms. The former group which is adjuvanted by mineral salts primarily gives lead to a Th2 response which is protective for the mentioned diseases, whereas similar adjuvantation could have an inhibitory effect on the immune response necessary for protection against the live attenuated vaccine components (E.B. Lindblad et al., Infect. Immun. 65(2):623-629 (1997)). This dichotomy currently limits the possibilities of making combination vaccines where diseases from the two groups, respectively, are represented. The diseases covered by the live attenuated vaccines are often requiring a Th1 type immune response as protecting against these diseases does not depend upon an antibody response alone.

There is still a need for further investigation in order to improve the immunogenicity of DNA vaccines. It is noted in this connection that as far as the inventors are aware the possibility of combining DNA nucleotide-based vaccines with conventional protein or adsorbed protein-based vaccines for establishing an efficient combination of these two technologies has hitherto not been explored, especially not in relation to the use of negatively-charged mineral-based adjuvants.

The DNA vaccine technology has the potential of inducing a Th1 type response necessary for protection against those diseases which are at present prevented by the group of live attenuated vaccines. The present invention provides a combination of at least one DNA vaccine with one or more classically adjuvanted Th2 stimulating vaccines, thereby overcoming such limitations of preparing an extended programme of combination vaccines.

### Summary of the invention

The present invention provides a combined DNA/Protein antigen vaccine composition suitable for administration to a vertebrate host, including man, which comprises:
(a) a polynucleotide vaccine component comprising at least one polynucleotide encoding at least one antigen, such that introduction of said formulation into said vertebrate host results in expression of a biologically effective amount of said antigen or antigens so as to induce a prophylactic or therapeutic immune response;
(b) a protein antigen vaccine component comprising at least one protein antigen selected from the group of model protein antigens and vaccine protein antigens; and
(c) a mineral-based, negatively charged adjuvant, further characterized in that said mineral-based, negatively charged adjuvant is pre-incubated or mixed with said at least one protein antigen vaccine component prior to being formulated with said polynucleotide vaccine component.

In a preferred embodiment of the invention said mineral-based, negatively charged adjuvant is an adjuvant selected from the group of aluminium phosphate-based and aluminium hydroxyphosphate-based adjuvants.

In another embodiment of the invention said mineral-based, negatively charged adjuvant is an adjuvant selected from the group of calcium phosphate-based and calcium hydroxyphosphate-based adjuvants.

As such, the present invention relates to a vaccine composition as described herein, wherein said mineral-based negatively charged adjuvant is an aluminium salt or a calcium salt.

Accordingly, the present invention relates to a vaccine composition as described herein, wherein said aluminium or calcium salt is selected from the group consisting of aluminium phosphate, aluminium hydroxyphosphate, phosphate-treated aluminium hydroxide, calcium phosphate, calcium hydroxyphosphate, and phosphate-treated calcium hydroxide.

Suitable polynucleotide vaccine components which can be used In the vaccine composition according to the invention include components from vaccines where the classical aluminium adjuvants are considered Inadequate to elicit protection, for example intracellular parasitic bacteria like Mycobacterium sp. and Leishmania sp., and viruses like cytomegalovirus (CMV), HTLV-I, HIV, hepatitis C and D, influenza, measels, rubella, and tumour-associated antigens.

Suitable model protein antigens may range from acidic IEP proteins, such as bovine serum albumin and human serum albumin, to basic IEP proteins, such as lysozyme.

Suitable vaccine protein antigens include but are not limited to detoxified toxins as well as other vaccine relevant antigens at present used in conventional vaccines against e.g. tetanus, diphtheria, botulinus poisoning, pertussis, inactivated poliomyelitis, hepatitis A and B, and the like.

As such, the present invention relates to a vaccine composition as described herein, wherein said group of vaccine protein antigens includes a surface protein or a core protein of HBV, a de-toxified toxin from the bacteria Clostridium tetani (*i.e*. tetanus toxoid), a de-toxified toxin from the bacteria Clostridium botulinus (*i.e*, botulinus toxoid), and a de-toxified toxin from the bacteria Corynebacterium diphtheriae (*i.e*. diphtheria toxoid).

Accordingly, the present invention relates to a vaccine composition as described herein, wherein said group of vaccine protein antigens includes protein antigens derived from inactivated poliovirus.

In a further aspect of the invention the use of a mineral-based, negatively charged adjuvant is provided as a component in a combined DNA/Protetn based vaccine composition as herein defined.

In another aspect of the invention a kit is provided comprising a vaccine composition as defined above, in a unit dose form for administration to a vertebrate recipient, including man.

In yet another aspect of the invention a method of inducing an immune response in a vertebrate host is provided which comprises introducing a vaccine composition as defined above into said vertebrate host.

As such, the present invention relates to a method for preparing a vaccine composition as described herein, wherein a mineral-based negatively charged adjuvant is preincubated or mixed with at least one protein antigen vaccine component prior to formulating with a polynucleotide vaccine component.

These and other embodiments of the present invention will be outlined in more detail in the following detailed description.

### Brief description of the drawing

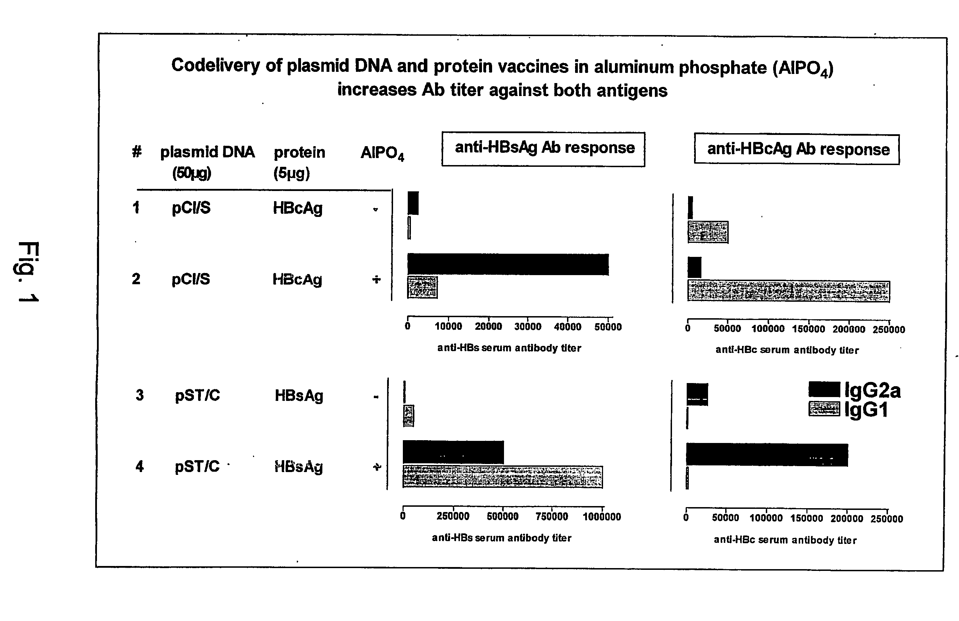

Figure 1 shows that the codelivery of plasmid DNA and protein vaccines in aluminum phosphate (AlPO₄) increases Ab titer against both antigens. BALB/c mice were vaccinated intramuscularly by single injection of the combination of 5 µg recombinant (HBcAg or HBsAg) particles and 50 µg plasmid DNA encoding HBsAg or HBcAg (pCl/S, pST/C). Vaccines containing either rHBcAg and pCl/S (group 1,2) or rHBsAg and pST/C (group 3,4) were delivered alone (group 1,3) or formulated with AlPO₄ adjuvant (group 2,4). Sera were obtained 4 weeks post-immunization and tested for anti-HBsAg (left panel) and anti-HBcAg (right panel) antibodies by ELISA. The mean antibody titers of 3 individual mice are shown.

### Definitions

The term "polynucleotide vaccine" (PNV) which is identical to "DNA vaccine", as used herein, is meant to indicate a DNA vector containing a gene encoding a viral, bacterial, parasitic or tumor antigen which has been shown to express that respective antigen subsequent to administration to the vertebrate, for example by intramuscular injection.

The term "model protein antigen" as used herein is meant to define a protein which is not derived from an infectious microorganism which may cause one or more diseases. Hence a model protein antigen is not included in a vaccine preparation with the aim of eliciting a protective immune response towards the model protein itself.

The term "conventional vaccines", as used herein, is meant to define well-established vaccines, whether they are based on (1) live, attenuated vaccine components (not adjuvanted), or (2) killed inactivated vaccine components, killed whole cells, purified subunit or peptide components (which are adjuvanted), using e.g. mineral adjuvants

The term "vaccine protein antigen" as used herein, refers to an antigen, regardless whether it is a protein-containing cell fragment, a purified protein, a synthetic peptide or whether in its nature it consists of amino acids only or of amino acids in combination with other biological molecules, like carbohydrates or lipids, derived from an infectious organism against which the vaccine is intented to protect. The purpose of including the vaccine protein antigen is to induce specific and protective immunity towards the infection caused by the organism that the vaccine protein relevant was derived from.

The term "adjuvant", as used herein, refers to one or more compounds that enhance the immune response against the antigenic components of the vaccine formulation or helps delivering the nucleotide or provides a stimulative effect for the immune response to transcription products of the injected DNA nucleotides.

The term "negatively charged mineral-based phosphate adjuvant", as used herein, refers to preformed aluminium phosphate or aluminium hydroxyphosphate gel adjuvants, and the like, which are characterized by having an acidic point of zero charge (PZC). It also refers to a formulation by which preformed aluminium hydroxide adjuvant (which normally has an alkaline PZC) after its formation is incubated with phosphate ions to form a complex with these by which process it also - subsequently - achieves an acidic PZC. It also refers to calcium phosphate or calcium hydroxyphosphate based adjuvants.

The term "point of zero charge" or "PZC", as used herein refers to the specific pH value at which a given mineral-based adjuvant has no net charge. As such the PZC is analogous to the isoelectric point (IEP) of a protein as known from protein chemistry. At pH values above the PZC the net charge of the mineral-based adjuvant is negative and below the PZC the net charge of the mineral-based adjuvant is positive.

### Detailed description of the invention

The present invention is based on the surprising finding, after extensive research and experimentation, that aluminium phosphate or alumium hydroxyphosphate-mediated enhancement of the immunogenicity of DNA vaccines was further improved by pre-incubating aluminium phosphate or aluminium hydroxyphosphate with at least one suitable protein. It was also found that aluminium phosphate and aluminium hydroxyphosphate and their calcium counterparts can be used to combine protein- and DNA-based vaccination to prime an enhanced and differentiated specific immunity.

The invention relates in one aspect to a novel vaccine formulation comprising nucleic acid molecules and a mineral-based, negatively charged adjuvant delivered in a biologically effective concentration so as to promote the effective induction of an immune response directed towards one or more specific antigens encoded by the nucleic acid molecule(s). According to this aspect the adjuvant, when delivered in conjunction, e.g. pre-incubated or pre-mixed with a suitable protein, seems to have a different appearance as compared to the original vaccine formulations on which this improvement is based which have been extensively described, *inter alia* in WO 00/2591 . Apart from the improvement defined above, any aspect of said disclosure including definitions, preparation of the pharmaceutical formulations, selection and amounts of ingredients, methods of inducing an immune response in an vertebrate host using the pharmaceutical formulations, way of delivery of the formulations to the vertebrate host, the diseases or disorders to be treated by the formulations, etc., apply equally to the present invention.

The present invention is further based on the surprising finding that the enhancement is mediated by the negatively charged mineral-based phosphate adjuvant (as exemplified by, but not limited to aluminium phosphate and aluminium hydroxy phosphate), with a suitable protein, as exemplified by either a model protein, such as an acidic IEP protein, for example bovine serum albumine (BSA) having low adsorption affinity, or a basic IEP protein, for example hen egg lysozyme (HEL) having high adsorption affinity, or, alternatively, a vaccine-relevant protein antigen, as exemplified by - but not limited to - two vaccine-relevant antigens of hepatitis B virus (HBV), i.e. its envelope protein (hepatitis B surface antigen, HBsAg) and its nucleoprotein (hepatitis B core antigen, HBcAg). Accordingly, a protein antigen, whether or not intended to raise protective immune responses against itself, can be suitably and advantageously co-delivered with DNA vaccines formulated in aluminium phosphate, aluminium hydroxyphosphate, or another suitable negatively charged mineral adjuvant.

It was further found that AlPO₄ is particularly suitable as an adjuvant to formulate a vaccine composition that delivers expression plasmid-encoded antigens together with recombinant protein antigens. The vaccine composition combines (i) the adjuvant effect of AlPO₄, (ii) the adjuvant effect of plasmid DNA, (iii) the enhanced immunogenic delivery of a DNA vaccine by AlPO₄, and (iv) the potent co-priming of multispecific, humoral and cellular immune responses to a variety of antigens that usually is achieved only by immunization with vaccines containing live, attenuated or killed pathogens.

When analyzing the priming of murine humoral and CTL responses to vaccine-relevant antigens formulated in aluminium-based adjuvants, no boosting injections were given to exclusively determine the potency of the vaccine formulations under study in priming CTL and T cell-dependent immune responses. (It will be understood that boosting injections are not excluded from the the present invention).

It is known that plasmid DNA and proteins with an acidic IEP adsorb efficiently onto Al(OH)₃, but not to AlPO₄ [J.B. Ulmer et al., Vaccine (1999) 18:18-28; S.J. Seeber et al., Vaccine (1991) 9:201-203]. An immunogenic delivery of DNA vaccines with concentional Al(OH)₃ adjuvant has not been achieved while Al(OH)₃ is widely used to deliver protein antigens in immunogenic form. Without being bound to any theory, the present inventors believe that the high content of phosphate in the DNA strand may give lead to the very high binding affinity to Al(OH)₃ and that this binding impairs the subsequent transcription of the protein antigen encoded by the DNA. A "depot effect", i.e. the slow release of antigen *in situ* combined with an immune-stimulating effect of the aluminium salt, has often been proposed to underlie the adjuvant activity. The lack of adsorption of plasmid DNA onto AlPO₄ (but not Al(OH)₃) seems to be an essential prerequisite for its ability to deliver DNA vaccines in an adequate and functional form. When low doses of a "naked" DNA vaccine are injected i.m. without adjuvants, specific priming of CTL, but not of serum antibody responses is observed [W. Böhm et al., J. Immunol. Methods (1996) 193:29-40]. Delivery of a DNA vaccine by AlPO₄ did not enhance its ability to prime specific, polarization of the response against the individual components of the vaccine. The antigens encoded by DNA vaccines would preferentially prime Th1 immunity while the antigens formulated into the AlPO₄ as recombinant proteins would preferentially induce Th2 immunity. This is clearly shown in Figure 1 where one can see that the DNA-derived antibody repsonse is biased towards the IgG2a subclass, indicative of Th1 immunity, whereas the protein-derived antibody response is biased towards the IgG1 subclass, indicative of Th2 immunity. This does not take into account intrinsic features of an antigen that can drive the polarization of the immune response they induce into a Th1 or Th2 bias independent of the locally prevailing cytokine milieu [P. Riedl et al., J. Immunol. (2002) 168:4951-4959; J. Reimann and R. Schirmbeck, Dev. Biol. (2000) 104:15-24]. However, the key point is that the vaccine delivery strategy according to the present invention supports the formulation of a single vaccine composed of very different types of vaccine constructs that prime *in situ* a diverse spectrum of immune responses differing in specificity and in the repertoire of specific effector functions they can mediate, i.e., a differentiated Th1 to the DNA encoded antigen, including a CTL response, and a Th2 response to the protein antigen.

Experimental vaccines were formulated in which low doses (1-5 µg/mouse) of antigen-encoding plasmid DNA together with low doses of either a model protein antigen (BSA), or a vaccine-relevant protein antigen (HBsAg or HBcAg) was mixed with aluminium phosphate or aluminium hydroxyde. The immune responses elicited by this combination vaccine against the protein antigen adsorbed to aluminium phosphate were enhanced in the presence of the DNA vaccine, and did not interfere with immune responses against the antigen encoded by the DNA vaccine.

To be more specific, low doses of different DNA vaccines formulated with AlPO₄ induced enhanced humoral responses and supported priming of MHC class I-restricted cellular immunity. Different proteins mixed with the plasmid DNA vaccine in the AlPO₄ formulation did not impair its immunogenicity. Co-injection of two different, vaccine-relevant antigens in the same AlPO₄ formulation, one as a DNA vaccine, the other as a recombinant protein, elicited polyvalent, humoral and cellular immune responses to all antigens delivered. The isotype profiles of the induced humoral responses and the cytokine profiles of the specifically primed T cell responses indicated that the combined vaccines supported co-priming of Th1- and Th2-biased, as well as differentiated responses.

Therefore, protein antigen-containing mineral-based, negatively charged adjuvants, such as aluminium phosphate and for example also aluminium hydroxyphosphate can efficiently enhance not only plasmid DNA vaccines but also provides a novel strategy for immunogenic, multivalent combined protein/DNA vaccine delivery.

The invention is further illustrated by the following examples which, however, are not intended to limit the invention in any respect.

### Experimental

Immunization of BALB/c mice with protein- or DNA-based vaccines was chosen as the preclinical model since a significant amount of the preclinical experience obtained with novel vaccination approaches has been generated in this species.

Standard immunization procedures were used based on the intramuscular injection of either 5, 50 or 100 µg plasmid DNA vaccines, or 0.5-5 µg protein antigen.

### 1. Mixing a model protein (antigen) together with a plasmid DNA vaccine to Al phosphate or Al hydroxyphosphate enhances the immunogenicity of the latter

BALB/c mice were immunized i.m. with 5, 50 or 100 µg pCI/S plasmid DNA (encoding the small HBsAg).

The DNA was delivered:
- without vehicle (as "naked" plasmid DNA)
- with aluminium phosphate
- without protein antigen added
- with (A) BSA or (B) HEL added

The antibody response was determined 4 weeks after a single immunization.

### (A) BSA system (see Table 1)

**Table 1**

| **group** | **pCI/S DNA vaccine (µg/mouse)** | **protein (BSA)** | **aluminium phosphate** | **anti-HBsAg serum antibody titer mlU/ml** |
|---|---|---|---|---|
| 1 | 5 | - | - | <10 |
| 2 | 50 | - | - | 978 |
| 3 | 100 | - | - | 1898 |
| | | | | |
| 4 | 5 | - | + | 458 |
| 5 | 50 | - | + | 2889 |
| | | | | |
| 6 | 5 | + | - | <10 |
| 7 | 50 | + | - | 2756 |
| | | | | |
| 8 | 5 | + | + | 1789 |
| 9 | 50 | + | + | 4572 |
| | | | | |
| 10 | - | + | - | <10 |

### (B) HEL system (see Table 2)

**Table 2**

| **group** | **pCI/S DNA vaccine (µg/mouse)** | **protein (HEL)** | **aluminium phosphate** | **anti-HBsAg serum antibody titer mlU/ml** |
|---|---|---|---|---|
| 1 | 5 | - | - | <10 |
| 2 | 50 | - | - | 613 |
| 3 | 100 | - | - | 965 |
| | | | | |
| 4 | 5 | - | + | 549 |
| 5 | 50 | - | + | 2790 |
| | | | | |
| 6 | 5 | + | - | <10 |
| 7 | 50 | + | - | 1473 |
| | | | | |
| 8 | 5 | + | + | 753 |
| 9 | 50 | + | + | 3317 |
| | | | | |
| 10 | - | + | - | <10 |

These results allow the following conclusions:
- A dose-dependent antibody response against a DNA nucleotide encoded antigen is induced by a single i.m. injection of the pCI/S DNA vaccine.
- The immunogenicity of the pCI/S DNA vaccine is enhanced when it is mixed before i.m. delivery with aluminium phosphate or alumium hydroxyphosphate.
- When protein antigens (BSA) are mixed with aluminium phosphate or alumium hydroxyphosphate the immunogenicity of the pCI/S DNA vaccine also mixed to aluminium phosphate or alumium hydroxyphosphate is strikingly enhanced.

### 2. Mixing a vaccine protein antigen and a plasmid DNA vaccine with Al phosphate

BALB/c mice were immunized i.m. with 5, 50 or 100 µg pCI/LC149 plasmid DNA (encoding a secreted, truncated variant of the HBV core antigen HBcAg).

The DNA vaccine was delivered:
- without vehicle (as 'naked' plasmid DNA)
- with aluminium phosphate
- without protein antigen added
- with HBsAg added

HBsAg (2 µg/mouse) was delivered.
- without vehicle (as 'naked' protein particles)
- with aluminium phosphate
- without protein antigen added
- with DNA vaccine added

The antibody response was determined 4 weeks after a single immunization. The results are shown in Table 3 below.

**Table 3**

| **group** | **pCI/LC₁₄₉ DNA vaccine (µg/mouse)** | **Protein antigen: HBsAg** | **aluminium phosphate** | **reciprocal anti-HBc/eAg endpoint antibody titer** | **anti-HBsAg serum antibody titer mlU/ml** |
|---|---|---|---|---|---|
| 1 | 5 | - | - | <10 | <10 |
| 2 | 50 | - | - | 10.000 | <10 |
| 3 | 100 | - | - | 26.000 | <10 |
| | | | | | |
| 4 | 5 | - | + | 2.500 | <10 |
| 5 | 50 | - | + | 75.000 | <10 |
| | | | | | |
| 6 | 5 | + | - | <10 | 2187 |
| 7 | 50 | + | - | 10.000 | 3210 |
| | | | | | |
| 8 | 5 | + | + | 12.500 | 2630 |
| 9 | 50 | + | + | 120.000 | 3540 |
| | | | | | |
| 10 | - | + | + | <10 | 2100 |

These results allow the following conclusions:
- A dose-dependent antibody responses is induced by a single i.m. injection of the pCI/LC₁₄₉ DNA vaccine; the immunogenicity of the DNA vaccine is enhanced when it is mixed before i.m. delivery with aluminium phosphate or aluminium hydroxyphosphate.
- Protein antigens (HBsAg) and DNA vaccines are mixed with aluminium phosphate or aluminium hydroxyphosphate prime polyvalent immune responses.

### 3. Adsorption of vaccine protein antigen (HBsAg) to Al phosphate / Al hydroxy-phosphate or Al hydroxide

### In vitro experiments:

i. 91 µl = 450 µg Al of either aluminium phosphate or aluminium hydroxide were incubated for 24 h at 4°C (under constant shaking) with 100, 50, 10, 5 or 1 µg HBsAg in a total volume of 200 µl; a parallel set of samples was incubated additionally with 50 µg plasmid DNA.
ii. the alum was spun down and supernatants were harvested.
iii. HBsAg (Abbott AxSym HBsAg V2-kit (cat.no. 7A 10-22, Abbott, Wiebaden, Germany) in supernatants (S/N) was measured by the Abbott ELISA.

The results are shown in the following graph.

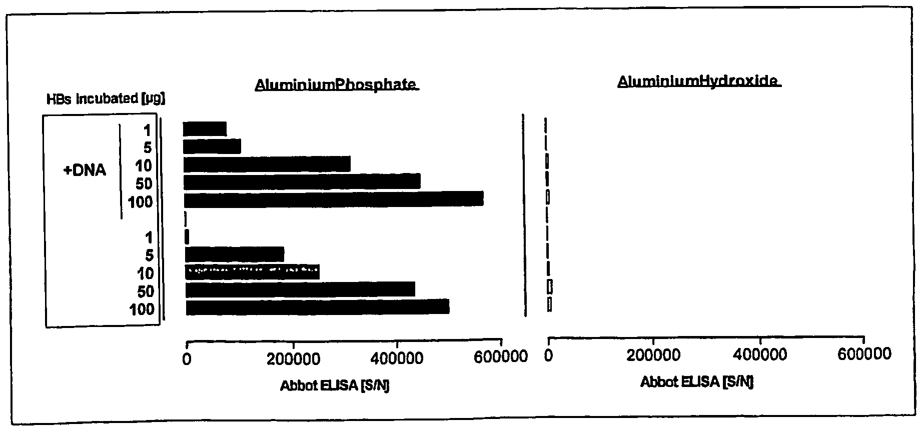

HBsAg was readily detected in S/N of mixtures of antigen with aluminium phosphate (even when as low amounts as only 1 µg HBsAg was mixed with the phosphate adjuvant) while no HBsAg was detectable after incubation with aluminium hydroxide. There was no difference in HBsAg release when plasmid DNA was also mixed (together with HBsAg protein) to the aluminium adjuvant preparation.

These results allow the following conclusions.
- Aluminium phosphate and alumium hydroxyphosphate do not adsorb HBsAg but aluminium hydroxide adsorbs HBsAg efficiently.
- Plasmid DNA in the applied quantities suitable for vaccination has no influence on the adsorption or non-adsorption of protein antigen to aluminium adjuvants.

## Claims

1. A vaccine composition suitable for administration to a vertebrate host, including man, which comprises:
(a) a polynucleotide vaccine component comprising at least one polynucleotide encoding at least one antigen, such that introduction of said formulation into said vertebrate host results in expression of a biologically effective amount of said antigen or antigens so as to induce a prophylactic or therapeutic immune response;
(b) a protein antigen vaccine component comprising at least one protein antigen selected from the group of model protein antigens and vaccine protein antigens; and
(c) a mineral-based, negatively charged adjuvant,
further **characterized in that** said mineral-based negatively charged adjuvant is preincubated or mixed with said at least one protein antigen vaccine component prior to formulating with said polynucleotide vaccine component.

2. A vaccine composition according to claim 1, wherein said mineral-based negatively charged adjuvant is an aluminium salt or a calcium salt.

3. A vaccine composition according to claim 2, wherein said aluminium or calcium salt is selected from the group consisting of aluminium phosphate, aluminium hydroxyphosphate, phosphate-treated aluminium hydroxide, calcium phosphate, calcium hydroxyphosphate, and phosphate-treated calcium hydroxide.

4. A vaccine composition according to any one of claims 1 to 3, wherein said group of model protein antigens range from acidic isoelectric point (IEP) proteins to alkaline IEP proteins.

5. A vaccine composition according to any one of claims 1 to 4, wherein said group of vaccine protein antigens includes a surface protein or a core protein of HBV, a de-toxified toxin from the bacteria Clostridium tetani, tetanus toxoid, a de-toxified toxin from the bacteria Clostridium botulinus, botulinus toxoid, and a de-toxified toxin from the bacteria Corynebacterium diphtheriae, diphtheria toxoid.

6. A vaccine composition according to any one of claims 1 to 4, wherein said group of vaccine protein antigens includes protein antigens derived from inactivated poliovirus.

7. A kit comprising a vaccine composition as defined in any one of the claims 1-6 in a unit dose form for administration to a vertebrate recipient, including man.

8. Method for preparing a vaccine composition according to any of claims 1 to 6, wherein a mineral-based negatively charged adjuvant is preincubated or mixed with at least one protein antigen vaccine component prior to formulating with a polynucleotide vaccine component.

## Patentansprüche

1. Impfstoffzusammensetzung, die zur Verabreichung an einen Wirbeltierwirt, einschließlich des Menschen, geeignet ist und Folgendes aufweist:
(a) eine Polynukleotidimpfstoffkomponente, welche mindestens ein Polynukleotid aufweist, das mindestens ein Antigen kodiert, derart, dass eine Einführung der Formulierung in den Wirbeltierwirt zur Expression einer biologisch wirksamen Menge des Antigens oder der Antigene führt, um eine prophylaktische oder therapeutische Immunreaktion zu induzieren;
(b) eine Proteinantigenimpfstoffkomponente, welche mindestens ein Proteinantigen aufweist, das aus der Gruppe von Modellproteinantigenen und Impfstoffproteinantigenen ausgewählt ist; und
(c) einen negativ geladenen Hilfsstoff auf Mineralbasis, weiter **dadurch gekennzeichnet, dass** der negativ geladene Hilfsstoff auf Mineralbasis vor der Formulierung mit der Polynukleotidimpfstoffkomponente mit der mindestens einen Proteinantigenimpfstoffkomponente vorinkubiert oder gemischt wird.

2. Impfstoffzusammensetzung nach Anspruch 1, wobei es sich bei dem negativ geladenen Hilfsstoff auf Mineralbasis um ein Aluminiumsalz oder Kalziumsalz handelt.

3. Impfstoffzusammensetzung nach Anspruch 2, wobei das Aluminium- oder Kalziumsalz aus der Gruppe ausgewählt ist, die aus Aluminiumphosphat, Aluminiumhydroxyphosphat, phosphatbehandeltem Aluminiumhydroxid, Kalziumphosphat, Kalziumhydroxyphosphat und phosphatbehandeltem Kalziumhydroxid besteht.

4. Impfstoffzusammensetzung nach einem der Ansprüche 1 bis 3, wobei die Gruppe von Modellproteinantigenen von Proteinen mit saurem isoelektrischem Punkt (IEP) bis hin zu Proteinen mit alkalischem IEP reicht.

5. Impfstoffzusammensetzung nach einem der Ansprüche 1 bis 4, wobei die Gruppe von Impfstoffproteinantigenen ein Oberflächenprotein oder ein Kernprotein von HBV, ein enttoxifiziertes Toxin aus dem Bakterium Clostridium tetani, Tetanustoxoid, ein enttoxifiziertes Toxin aus dem Bakterium Clostridium botulinus, Botulinustoxoid, und ein enttoxifiziertes Toxin aus dem Bakterium Corynebacterium diphtheriae, Diphtherietoxoid, enthält.

6. Impfstoffzusammensetzung nach einem der Ansprüche 1 bis 4, wobei die Gruppe von Impfstoffproteinantigenen Proteinantigene enthält, die von inaktivierten Poliovirus stammen.

7. Kit, welches eine Impfstoffzusammensetzung aufweist, wie in einem der Ansprüche 1-6 definiert, in einer Einheitsdosisform zur Verabreichung an einen Wirbeltierempfänger, einschließlich des Menschen.

8. Verfahren zum Zubereiten einer Impfstoffzusammensetzung nach einem der Ansprüche 1bis 6, wobei ein negativ geladener Hilfsstoff auf Mineralbasis vor einer Formulierung mit einer Polynukleotidimpfstoffkomponente mit mindestens einer Proteinantigenimpfstoffkomponente vorinkubiert oder gemischt wird.

## Revendications

1. Composition vaccinale adaptée à l'administration à un hôte vertébré, y compris un être humain, qui comprend :
(a) un composant vaccinal polynucléotide qui comprend au moins un polynucléotide codant pour au moins un antigène, de telle sorte que l'introduction de ladite formulation dans ledit hôte vertébré résulte en l'expression d'une quantité biologiquement efficace dudit antigène ou desdits antigènes pour induire une réponse immunitaire prophylactique ou thérapeutique ;
(b) un composant vaccinal antigène protéique qui comprend au moins un antigène protéique choisi dans le groupe constitué par des modèles d'antigènes protéiques et des antigènes protéiques vaccinaux ; et
(c) un adjuvant à charge négative à base de minéraux, en outre **caractérisée en ce que** ledit adjuvant à charge négative à base de minéraux est préincubé ou mélangé avec ledit ou lesdits composant(s) vaccinal (vaccinaux) antigène protéique avant la formulation avec ledit composant vaccinal polynucléotide.

2. Composition vaccinale selon la revendication 1, ledit adjuvant à charge négative à base de minéraux étant un sel d'aluminium ou un sel de calcium.

3. Composition vaccinale selon la revendication 2, ledit sel d'aluminium ou de calcium étant choisi dans le groupe constitué par le phosphate d'aluminium, l'hydroxyphosphate d'aluminium, l'hydroxyde d'aluminium traité au phosphate, le phosphate de calcium, hydroxyphosphate de calcium, et l'hydroxyde de calcium traité au phosphate.

4. Composition vaccinale selon l'une quelconque des revendications 1 à 3, ledit groupe de modèles d'antigènes protéiques se trouvant dans la plage de protéines ayant un point isoélectrique (PIE) acide à des protéines ayant un PIE alcalin.

5. Composition vaccinale selon l'une quelconque des revendications 1 à 4, ledit groupe d'antigène protéiques vaccinaux comprenant une protéine de surface ou une protéine core du HBV, une toxine détoxifiée de bactéries Clostridium tetani, l'anatoxine tétanique, une toxine détoxifiée de bactéries Clostridium botulinus, l'anatoxine botulique, et une toxine détoxifiée de bactéries Corynebacterium diphtheriae, l'anatoxine diphtérique.

6. Composition vaccinale selon l'une quelconque des revendications 1 à 4, ledit groupe d'antigènes protéiques vaccinaux comprenant des antigènes protéiques dérivés d'un poliovirus inactivé.

7. Kit comprenant une composition vaccinale selon l'une quelconque des revendications 1 à 6, sous une forme posologique unitaire pour l'administration à un receveur vertébré, y compris un être humain.

8. Procédé de préparation d'une composition vaccinale selon l'une quelconque des revendications 1 à 6, un adjuvant à charge négative à base de minéraux étant préincubé ou mélangé avec ledit ou lesdits composant(s) vaccinal (vaccinaux) antigène protéique avant la formulation avec un composant vaccinal polynucléotide.
